(19) Europäisches Patentamt
European Patent Office
Office européen des brevets

(11) **EP 3 276 348 A1**

(12) **EUROPEAN PATENT APPLICATION**

(43) Date of publication:
**31.01.2018 Bulletin 2018/05**

(51) Int Cl.:
***G01N 33/36*** (2006.01)

(21) Application number: **17182970.8**

(22) Date of filing: **25.07.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.07.2016 JP 2016148794**

(71) Applicant: **Murata Machinery, Ltd.
Kyoto-shi,
Kyoto 601-8326 (JP)**

(72) Inventor: **Oka, Masaki
Kyoto, 612-8686 (JP)**

(74) Representative: **Hoffmann Eitle
Patent- und Rechtsanwälte PartmbB
Arabellastraße 30
81925 München (DE)**

(54) **YARN ANALYZING DEVICE AND SPINNING MACHINE**

(57)     An analyzing device (60) is configured to analyze yarn (Y) to be processed in a fiber processing unit (2) based on yarn thickness information on thickness of the yarn (Y), the analyzing device including: an acquiring unit (60A) configured to acquire the yarn thickness information; an analyzing unit (60B) configured to analyze time variation of the yarn thickness based on the yarn thickness information acquired by the acquiring unit; and a display control unit (60C) configured to control a display unit (52) to display a graph in a result displaying area (Rl) having one of an abscissa (AX) and an ordinate (AY) for representing a frequency or a period in which a predetermined yarn thickness appears, the other of the abscissa and the ordinate for representing a distribution quantity of the frequency or the period, and at least one specific region (Ba, Bb, Bc, Bd, Be, Bf, Bg, Bh, Bi, Bx, By, Bz) in which a region corresponding to the frequency or the period in a specific range is displayed in a visually distinguishable manner, the graph representing a relation between the frequency or the period and the distribution quantity, the relation being analyzed by the analyzing unit.

*Fig.3*

**Description**

TECHNICAL FIELD

The present disclosure relates to an analyzing device and a spinning machine for yarn to be processed in textile machinery.

BACKGROUND

Spinning devices each including a yarn-defect detection device configured to measure the thickness of yarn to detect a defect of the yarn and each configured to cut the yarn on the basis of information on the thickness of the yarn detected by the yarn-defect detection device are known. The defect of the yarn herein means a yarn defect such as a slub, indicating an abnormal part where yarn thickness varies. An analyzing device (abnormal spindle-identifying system) is disclosed (see Japanese Unexamined Patent Publication No. 2007-224452, for example) that can identify an abnormal part by storing such information on yarn thickness to perform frequency analysis on the information, and by identifying a period in which a yarn defect occurs (hereinafter called "period or frequency of the yarn defect").

SUMMARY

In the conventional analyzing device described above, a result of performing frequency analysis on the information on yarn thickness is displayed on a screen. However, this result is merely information indicating the frequency (period) of the defect, and thus in which part abnormality, for example, has occurred cannot be identified only by glancing the information. In view of this, an operator who has viewed the screen needs to conduct an evaluation based on his/her experience. Consequently, evaluation results vary depending on differences in experience among operators, which may cause a situation in which the abnormal part cannot be identified and the abnormal part is left unhandled. The term "operator" herein means not only a worker who is allowed to perform only a predetermined work or operation for textile machinery, but also a worker (maintenance technician) who is authorized to perform general setting changes on operation and maintenance of the textile machinery.

In view of the foregoing, the present disclosure aims to provide an analyzing device and a spinning machine that can identify an abnormal part regardless of differences in experience among operators.

An analyzing device according to one aspect of the present disclosure is an analyzing device configured to analyze yarn to be processed in a fiber processing unit based on yarn thickness information on thickness of the yarn. The analyzing device includes: an acquiring unit configured to acquire the yarn thickness information; an analyzing unit configured to analyze time variation of the yarn thickness based on the yarn thickness information acquired by the acquiring unit; and a display control unit configured to control a display to display at least one graph in a result displaying area having one of an abscissa and an ordinate for representing a frequency or a period in which a predetermined yarn thickness appears, the other of the abscissa and the ordinate for representing the distribution quantity of the frequency or the period, and at least one specific region in which a region corresponding to the frequency or the period in a specific range is displayed in a visually distinguishable manner, the graph representing a relation between the frequency or the period and the distribution quantity, the relation being analyzed by the analyzing unit.

In the analyzing device thus configured, the relation between the frequency or the period and the distribution quantity analyzed by the analyzing unit is displayed as a graph in the result displaying area having the specific region in which the region corresponding to the frequency or the period in the specific range is displayed in a visually distinguishable manner. This enables an operator to determine, at a glance, which frequency region (periodic region) a frequency (period) with a projecting distribution quantity corresponds to. For example, when a relation between a frequency region (periodic region) and an abnormal part (e.g., a drafting roller) is derived from empirical rules, theoretical values, and the like, setting this frequency region (periodic region) as the specific region that is displayed in a visually distinguishable manner enables the operator to identify the abnormal part only by checking which specific region the region with a projecting distribution quantity belongs to. Consequently, regardless of differences in experience among operators, the abnormal part can be identified.

The term "graph" herein means a diagram representing a relation between two quantities or among two or more quantities that correspond to each other. The specific region displayed in the result displaying area may be provided singly or in plurality. When the specific region is provided in plurality, a plurality of abnormalities can be easily identified. The number of specific regions may be four or more.

In the analyzing device according to one aspect of the present disclosure, the display control unit may control the display unit so as to display at least one of a graph representing the distribution quantity for each frequency or each period and a graph representing the distribution quantity for each frequency band that is a predetermined range of the frequency or for each periodic band that is a predetermined range of the period. The graph representing the distribution quantity for each frequency or each period can provide data for identifying a mechanical factor, and the graph representing the distribution quantity for each frequency band or each periodic band can provide data for identifying a factor derived from yarn physical properties.

In the analyzing device according to one aspect of the present disclosure, the display control unit may control the display unit such that a portion in which the distribution quantity exceeds a predetermined threshold becomes visually distinguishable from a portion in which the distribution quantity does not exceed the predetermined threshold. This analyzing device can effectively notify the operator of a location where a problem may have occurred.

In the analyzing device according to one aspect of the present disclosure, the display control unit may control the display unit such that a portion in which the distribution quantity exceeds a predetermined threshold with respect to the distribution quantity in an adjacent frequency band or an adjacent periodic band becomes visually distinguishable from a portion in which the distribution quantity does not exceed the predetermined threshold. This analyzing device can effectively notify the operator of a location where a problem may have occurred.

In the analyzing device according to one aspect of the present disclosure, the display control unit may receive selection of the specific region made by operation of an operator. This analyzing device can manage various types of information in association with the specific region.

In the analyzing device according to one aspect of the present disclosure, the display control unit may control the display unit so as to display a first result displaying area and a second result displaying area as the result displaying area, display a first graph representing the distribution quantity for each frequency or each period in the first result displaying area, display a second graph representing the distribution quantity for each frequency band or each periodic band in the second result displaying area, and having received selection of one specific region contained in the result displaying area in which one of the first graph and the second graph is displayed, make visually distinguishable at least one specific region contained in the result displaying area in which the other of the first graph and the second graph is displayed, the at least one specific region being associated with the one specific region. This analyzing device can determine the abnormal part from a comprehensive viewpoint considering two factors of a mechanical factor and a factor derived from yarn physical properties.

In the analyzing device according to one aspect of the present disclosure, the display control unit may control the display unit such that, when selection of the specific region has been received, information on a part to become a factor of abnormality that occurs in the frequency or the period in the specific range corresponding to the specific region becomes visually distinguishable. This analyzing device enables the operator to know the abnormal part without referring to, for example, a list that represents a relation between each specific region and the corresponding abnormal part.

In the analyzing device according to one aspect of the present disclosure, the graph may be a bar graph. This analyzing device can display the relation between the frequency or the period and the distribution quantity in an easily understandable manner.

In the analyzing device according to one aspect of the present disclosure, the display control unit may control the display unit so as to display a bar of the bar graph representing the distribution quantity for each frequency or each period. This analyzing device can provide data for identifying a mechanical factor.

In the analyzing device according to one aspect of the present disclosure, the display control unit may control the display unit such that the bar having a distribution quantity exceeding a predetermined threshold becomes visually distinguishable from the bar having a distribution quantity not exceeding the predetermined threshold. This analyzing device can effectively notify the operator of a location where a problem may have occurred.

In the analyzing device according to one aspect of the present disclosure, the display control unit may control the display unit so as to display a bar of the bar graph representing the distribution quantity for each frequency band or each periodic band. This analyzing device can provide data for identifying a factor derived from yarn physical properties.

In the analyzing device according to one aspect of the present disclosure, the display control unit may control the display unit such that a bar having a protruding amount relative to the distribution quantity in an adjacent frequency band or an adjacent periodic band exceeding a predetermined threshold becomes visually distinguishable from the bar having a protruding amount not exceeding the predetermined threshold. This analyzing device can effectively notify the operator of a location where a problem may have occurred.

In the analyzing device according to one aspect of the present disclosure, the display control unit may control the display unit such that the specific region is displayed in a display mode different from that of the other region. This analyzing device enables the operator to easily distinguish the region corresponding to the frequency or the period in the specific range. In other words, in both cases when abnormality has occurred and when abnormality has not occurred, the specific region can be easily recognized. This analyzing device can effectively notify the operator of a location where a problem may have occurred.

In the analyzing device according to one aspect of the present disclosure, the display control unit may control the display unit such that the specific region is displayed in a color different from that of the other region. This analyzing device can effectively notify the operator of a location where a problem may have occurred. In addition to cases in which only differences in tint (hue), such as red, blue and green, are differentiated, the expression "is displayed in a different color" herein expresses a concept including cases in which only the degree of color lightness (brightness), such as black, dark gray and light gray, is differentiated, and also cases in which a combination of hue and brightness are differentiated.

In the analyzing device according to one aspect of the present disclosure, the specific region may include at least a region corresponding to a frequency or a period corresponding to a rotation period of a front top roller that is calculated based on a radius of the front top roller and a rotation speed of the front top roller. This analyzing device can provide data for identifying a malfunction that may occur due to a mechanical factor in a drafting device.

A spinning machine according to one aspect of the present disclosure includes: a drafting device configured to draft a fiber bundle; an air spinning device configured to generate yarn by twisting the fiber bundle drafted by the drafting device; a winding device configured to wind the yarn generated by the air spinning device to form a package; the aforementioned analyzing device; and a display unit. The display unit displays a graph in the result displaying area, the graph representing a relation between the frequency or the period and the distribution quantity, the relation being analyzed by the analyzing unit.

In the spinning machine thus configured, the relation between the frequency or the period and the distribution quantity analyzed by the analyzing unit is displayed as a graph in the result displaying area having the specific region in which the region corresponding to the frequency or the period in the specific range is displayed in a visually distinguishable manner. This enables the operator to determine, at a glance, which frequency region (periodic region) a frequency (period) the projecting distribution quantity correspondsto. For example, when a relation between a frequency region (periodic region) and an abnormal part (e.g., a drafting roller) is derived from empirical rules, theoretical values, and the like, setting this frequency region (periodic region) as the specific region that is displayed in a visually distinguishable manner enables the operator to identify the abnormal part only by checking which specific region the region with the projecting distribution quantity belongs to. Consequently, regardless of differences in experience among operators, the abnormal part can be identified.

According to the present disclosure, regardless of differences in experience among operators, the abnormal part can be identified.

## BRIEF DESCRIPTION OF THE DRAWINGS

FIG. 1 is a front view of a spinning machine according to one embodiment;
FIG. 2 is a functional block diagram illustrating a functional configuration of the spinning machine in FIG. 1;
FIG. 3 illustrates one example of display of a first bar graph;
FIG. 4 illustrates one example of display of a second bar graph;
FIG. 5 is a diagram in which part of the first bar graph in FIG. 3 is illustrated in an enlarged manner;
FIG. 6 illustrates one example of a pop-up display;
FIG. 7 is a diagram in which part of the second bar graph in FIG. 4 is illustrated in an enlarged manner;
FIG. 8 illustrates one example of a pop-up display;
FIG. 9 illustrates an example of display of a first bar graph and a second bar graph according to a modification 1;
FIG. 10A illustrates one example of a first bar graph in which the range of frequencies in the abscissa is 0 Hz to 200 Hz;
FIG. 10B illustrates one example of the first bar graph in which the range of frequencies in the abscissa is 0 Hz to 10 Hz;
FIG. 11 illustrates one example of a second bar graph according to a modification;
FIG. 12 illustrates one example of a second bar graph according to another modification; and
FIG. 13 illustrates an example of display of a first bar graph and a second bar graph according to still another modification.

## DETAILED DESCRIPTION

One embodiment will now be described with reference to the drawings. In the description of the drawings, like elements are designated by like numerals, and duplicate description is omitted.

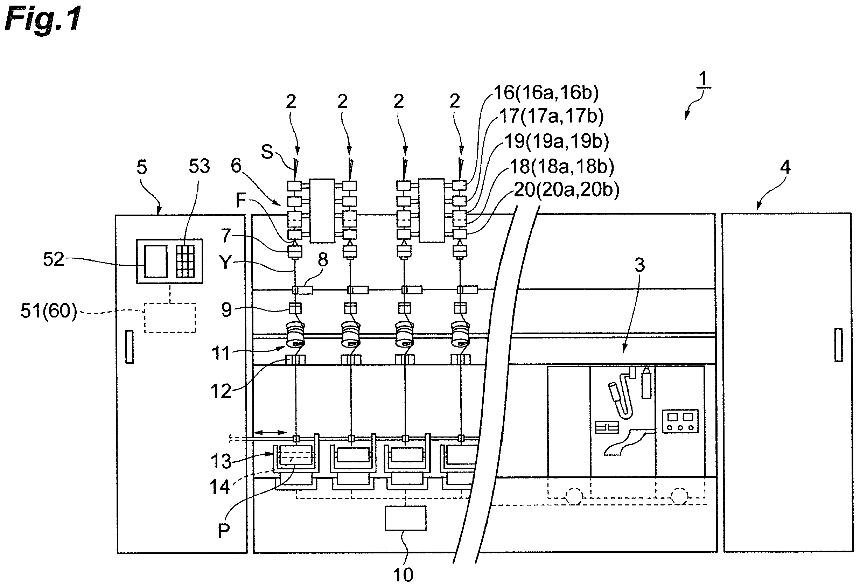

The following describes a spinning machine 1 with an analyzing device 60 according to the embodiment. As depicted in FIG. 1, the spinning machine 1 includes a plurality of spinning units 2, a splicing carrier 3, a doffing carrier (not depicted), a first end frame 4, and a second end frame 5. The spinning units 2 are aligned in a row. Each spinning unit 2 generates yarn Y and winds the yarn into a package P. When the yarn Y is cut or breaks in a spinning unit 2 for any reason, the splicing carrier 3 splices the yarn Y in the spinning unit 2. When the package P becomes a full roll in a spinning unit 2, the doffing carrier doffs the package P and supplies a new bobbin 14 to the spinning unit 2.

Each spinning unit 2 includes, in the order from the upstream side in a direction in which the yarn Y travels, a drafting device 6, an air spinning device 7, a yarn monitoring device 8, a tension sensor 9, a yarn storage device 11, a waxing device 12, and a winding device 13. However, the unit controller 10 may be provided for each of the spinning units 2.

The drafting device 6 drafts a sliver (fiber bundle) S. The air spinning device 7 generates the yarn Y by twisting the fiber bundle F drafted by the drafting device 6 using swirling airflow. The drafting device 6 has a plurality of pairs of drafting rollers 16, 17, 19, and 20 that are disposed along a direction in which the sliver S is fed (conveying direction), and uses these drafting rollers 16, 17, 19, and 20 to draft the fiber bundle F. The drafting device 6 includes, as the drafting rollers 16, 17, 19, and 20, back rollers 16, third rollers 17, middle rollers 19 around which apron belts 18 are each wound, and front rollers 20. The drafting rollers 16, 17, 19, and 20 are top rollers 16a, 17a, 19a, and 20a and bottom rollers 16b, 17b, 19b, and 20b, respectively provided in pair. The top rollers 16a, 17a, 19a, and 20a and the bottom rollers 16b, 17b, 19b, and 20b are disposed so as to sandwich the sliver S therebetween. The apron belts 18 are a top apron belt 18a and a bottom apron belt 18b.

The drafting device 6 includes motors configured to drive the respective bottom rollers of the drafting rollers 16, 17, 19, and 20. In the present embodiment, one motor drives and rotates one drafting roller, but one motor may drive and rotate a plurality of drafting rollers. For example, the back rollers 16 and the third rollers 17 that are low-speed drafting rollers may be both driven and rotated by one motor. Alternatively, one or adjacent two back bottom rollers 16b may be driven by one motor, one or adjacent two third bottom rollers 17b may be driven by one motor, middle bottom rollers 19b of all spinning units 2 or a predetermined number of spinning units 2 may be simultaneously driven and rotated by one motor, and front bottom rollers 20b of all spinning units 2 or a predetermined number of spinning units 2 may be simultaneously driven and rotated by one motor.

The yarn storage device 11 eliminates slack in the yarn Y between the air spinning device 7 and the winding device 13. The waxing device 12 applies wax to the yarn Y between the yarn storage device 11 and the winding device 13. The winding device 13 winds the yarn Y onto the bobbin 14 to form the package P.

The yarn monitoring device 8 monitors information on traveling yarn Y between the air spinning device 7 and the yarn storage device 11 to detect the presence or absence of a yarn defect on the basis of themonitoredinformation. When having detected a yarn defect, the yarn monitoring device 8 transmits a yarn defect detection signal to the unit controller 10. The tension sensor 9 measures the tension of traveling yarn Y between the air spinning device 7 and the yarn storage device 11, and transmits a tension measurement signal to the unit controller 10. When the unit controller 10 determines that abnormality has occurred based on at least one of a result of detection by the yarn monitoring device 8 and a result of detection by the tension sensor 9, the yarn Y is cut in the spinning unit 2.

The unit controller 10 is provided in every predetermined number of spinning units 2, and controls operations of the spinning units 2. The unit controller 10 includes a central processing unit (CPU), a read only memory (ROM), a random access memory (RAM), and an input/output port, which are not depicted. The ROM stores therein a program for controlling the respective components of the spinning units 2. The CPU can perform processing for controlling the respective components of the spinning units 2 by reading a winding program stored in the ROM into the RAM and executing the winding program. The input/output port included in each unit controller 10 is connected to the machine control device 51, and is configured to be able to transmit and receive information therethrough.

Each unit controller 10 in the present embodiment is electrically connected to the yarn monitoring devices 8, and receives information on yarn thickness (apparent yarn thickness) output from each yarn monitoring device 8 to determine whether periodic yarn irregularities (periodic irregularities) are formed in the corresponding yarn Y. In each spinning unit 2, based on the presence or absence of periodic irregularities, cutting of the corresponding yarn Y is controlled. The unit controller 10 outputs the information on yarn thickness to the machine control device 51 described later in detail.

The first end frame 4 accommodates, for example, a collection device configured to collect fiber waste, yarn waste, and the like generated in the spinning unit 2. The second end frame 5 accommodates, for example, an air supply unit configured to adjust air pressure of compressed air (air) supplied to the spinning machine 1 and supply the air to the respective units in the spinning machine 1 and a driving motor configured to supply power to the respective units in the spinning unit 2.

The second end frame 5 includes the machine control device 51, a display device (display unit) 52, and an input key 53. The machine control device 51 intensively manages and controls the respective units of the spinning machine 1. The display device 52 can display, for example, information on at least the settings or the status of the spinning unit 2. An operator can set the spinning unit 2 by performing necessary operations through the input key 53.

The machine control device 51 includes a CPU, a RAM, a ROM, and an input/output port. The ROM stores therein a control program for controlling the spinning units 2. The CPU can control at least one of each component of the spinning units 2 and a shared device by reading the control program stored in the ROM into the RAM and executing the control program.

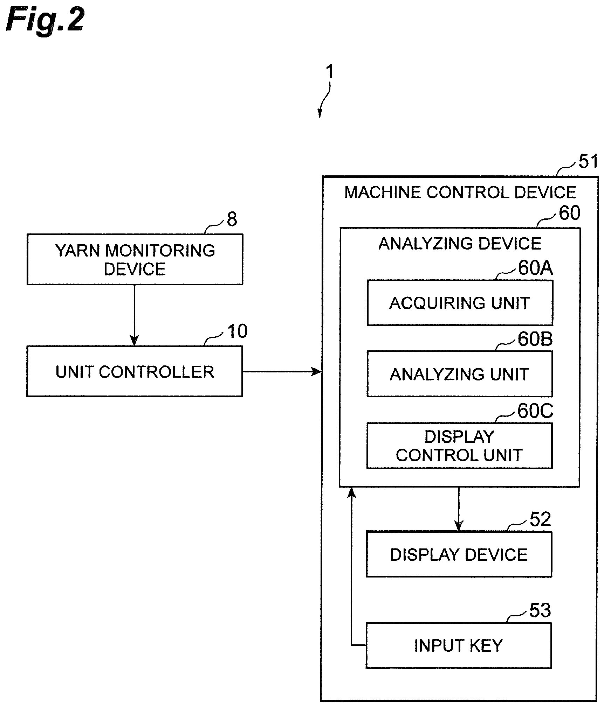

The analyzing device 60 analyzes yarn Y to be processed in each spinning unit 2 on the basis of yarn thickness information of the yarn Y. The analyzing device 60 is a circuit configured to analyze yarn Y to be processed in each spinning unit 2 on the basis of yarn thickness information of the yarn Y. The analyzing device 60 in the present embodiment is structured as the machine control device 51. Specifically, the machine control device 51 (hereinafter called "analyzing device 60") as the analyzing device 60 can cause an acquiring unit 60A, an analyzing unit 60B, and a display control unit 60C to function as depicted in FIG. 2 by cooperative operation of the hardware and the software described above.

The acquiring unit 60A acquires the yarn thickness information. Specifically, the acquiring unit acquires information on yarn thickness output from the unit controller 10, and stores therein the information. The acquiring unit 60A is a circuit configured to acquire information on yarn thickness output from the unit controller 10 and stores therein the information. The unit controller 10 acquires the information on yarn thickness from the yarn monitoring device 8.

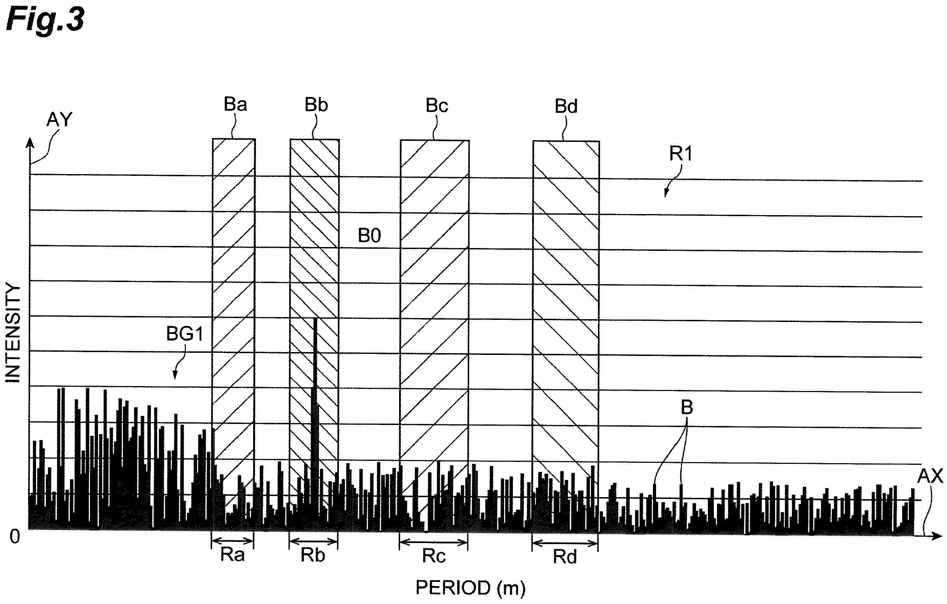

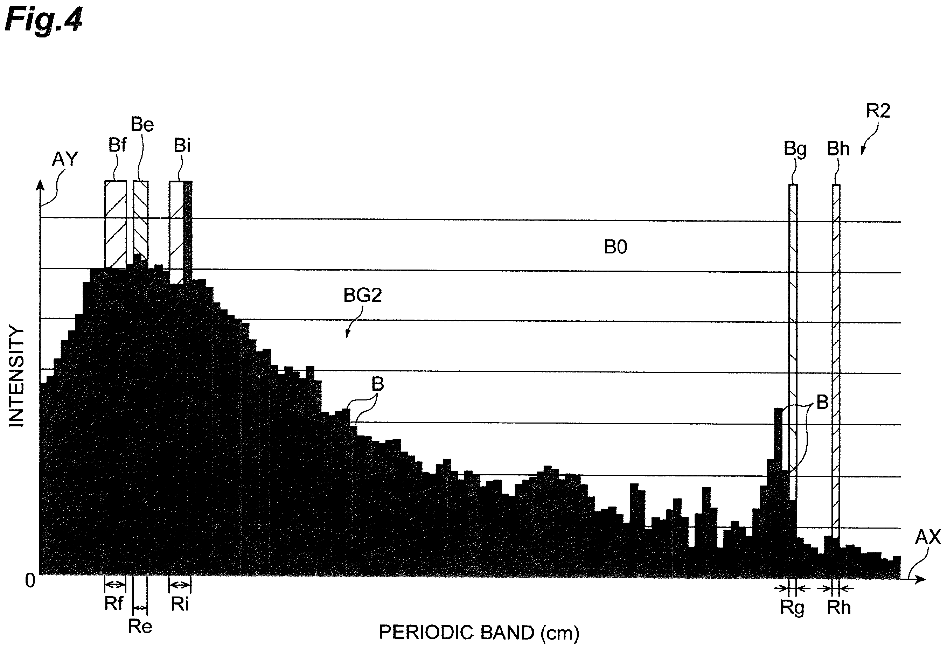

The analyzing unit 60B analyzes time variation of yarn thickness obtained from the yarn thickness information. The analyzing unit 60B is a device or a circuit configured to analyze time variation of yarn thickness obtained from the yarn thickness information. More specifically, the analyzing unit is a device or a circuit configured to acquire yarn thickness information, analyze the time variation of yarn thickness on the basis of the acquired yarn thickness information, and control the display unit to display a graph in a result displaying area having one of an abscissa and an ordinate for representing a frequency or a period in which a predetermined yarn thickness appears, the other of the abscissa and the ordinate for representing a distribution quantity of the frequency or the period, and at least one specific region in which a region corresponding to frequencies or periods in a specific range is displayed in a visually distinguishable manner. The graph represents a relation between the frequency or the period and a distribution quantity is displayed in a result displaying area, and the relation is identified by the analysis of the time variation. Specifically, the analyzing unit performs frequency analysis on the information on yarn thickness output from the unit controller 10. The analyzing unit 60B, as the frequency analysis, performs fast Fourier transformation (FFT) computation, for example, transforms the time axis into a period, and generates data representing a first bar graph (graph/first graph) BG1 depicted in FIG. 3. In other words, the analyzing unit 60B generates data for outputting a spectrogram based on the information on yarn thickness output from the unit controller 10. The analyzing unit 60B, as the frequency analysis, performs the fast Fourier transformation (FFT) computation and bandpass filtering, for example, and generates data with which a second bar graph (graph/second graph) BG2 depicted in FIG. 4 can be displayed.

As depicted in FIG. 3, the display control unit 60C controls the display device 52 such that a first bar graph BG1 representing with bars B a relation between periods and peak intensities (intensity) (CV%) (distribution quantities) corresponding to the respective periods is displayed in an overlapping manner in a result displaying area R1. The result displaying area has an abscissa AX for representing the periods, an ordinate AY for representing the peak intensities, and specific regions Ba, Bb, Bc, and Bd that are regions corresponding to periods in specific ranges and are displayed in a visually distinguishable manner. The relation is a result of analysis performed by the analyzing unit 60B. The display control unit 60C is a circuit configured to control the display device 52 such that the first bar graph BG1 representing with the bars B the relation between periods and peak intensities (intensity) (CV%) (distribution quantities) corresponding to the respective periods, which is a result of analysis performed by the analyzing unit 60B, is displayed in an overlapping manner in the result displaying area R1 having the abscissa AX for representing the periods, the ordinate AY for representing the peak intensities, and the specific regions Ba, Bb, Bc, and Bd that are regions corresponding to periods in specific ranges and are displayed in a visually distinguishable manner.

Herein, the expression "the first bar graph BG1 is displayed in an overlapping manner in the result displaying area R1" means that the first bar graph BG1 is displayed so as to be aligned with the scales of the abscissa AX and the ordinate AY displayed in the result displaying area R1. CV% is a "yarn-thickness uniformity ratio (yarn-irregularity uniformity ratio) ", which is one of indices representing the quality of yarn, is a value obtained by dividing the standard deviation of yarn thickness by the average, and is expressed by the following equation.

$$\mathrm{CV(\%) = standard\ deviation\ of\ yarn\ thickness \div average\ of\ yarn}$$

$$\mathrm{thickness \times 100(\%)}$$

For example, it is assumed herein that the following items are known based on empirical rules, theoretical values, and the like.

- When a value equal to or larger than a predetermined threshold appears in a period that belongs to a periodic range Ra, abnormality may have occurred in the front rollers 20.

- When a value equal to or larger than a predetermined threshold appears in a period that belongs to a periodic range Rb, abnormality may have occurred in the middle rollers 19.

- When a value equal to or larger than a predetermined threshold appears in a period that belongs to a periodic range Rc, abnormality may have occurred in the third rollers 17.

- When a value equal to or larger than a predetermined threshold appears in a period that belongs to a periodic range Rd, abnormality may have occurred in the back rollers 16.

These thresholds are values that are determined in advance for the respective periods.

The display control unit 60C causes the specific regions Ba, Bb, Bc, and Bd to be displayed in colors different from that of the other region (background) B0 excluding the specific regions Ba, Bb, Bc, and Bd. The specific regions Ba, Bb, Bc, and Bd may be displayed in colors different from each other, or may be displayed in the same color.

Such coloring caused by the display control unit 60C enables the operator to objectively recognize, for example, that abnormality may have occurred in the front rollers 20 when the specific region Ba containing the periodic range Ra overlaps a bar B that represents a peak intensity exceeding the predetermined threshold. In the same manner, the operator can objectively recognize, for example, that abnormality may have occurred in the middle rollers 19 when the specific region Bb containing the periodic range Rb overlaps a bar B representing a peak intensity exceeding the predetermined threshold, that abnormality may have occurred in the third rollers 17 when the specific region Bc containing the periodic range Rc a bar B representing a peak intensity exceeding the predetermined threshold, and that abnormality may have occurred in the back rollers 16 when the specific region Bd containing the periodic range Rd overlaps a bar B representing a peak intensity exceeding the predetermined threshold.

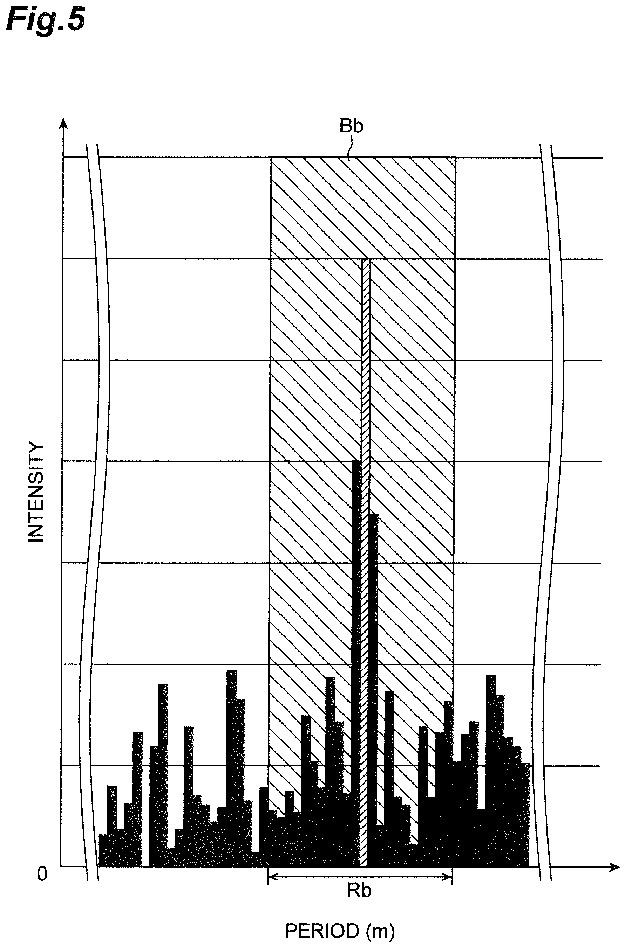

Furthermore, the display control unit 60C causes a bar B the peak intensity of which exceeds the predetermined threshold to be displayed in a manner visually distinguishable from bars B the peak intensities of which do not exceed the predetermined thresholds. For example, as depicted in FIG. 5, the display control unit 60C causes a bar B the peak intensity of which exceeds the predetermined threshold to be displayed in a color (hatched in FIG. 5) different from that of bars B the peak intensities of which do not exceed the predetermined thresholds. Over the result displaying area R1, a graph of the thresholds described above (i.e., thresholds for the respective periods) may be displayed.

The term "colors" used in the description herein contains hue, brightness, and a combination of hue and brightness. For example, the display control unit 60C may control the display device 52 such that the different specific regions Ba, Bb, Bc, and Bd are displayed in hues (or brightnesses) different from each other, and the specific region Ba, Bb, Bc, or Bd containing a distribution quantity exceeding the predetermined threshold is displayed in a brightness (or a hue) that is further changed.

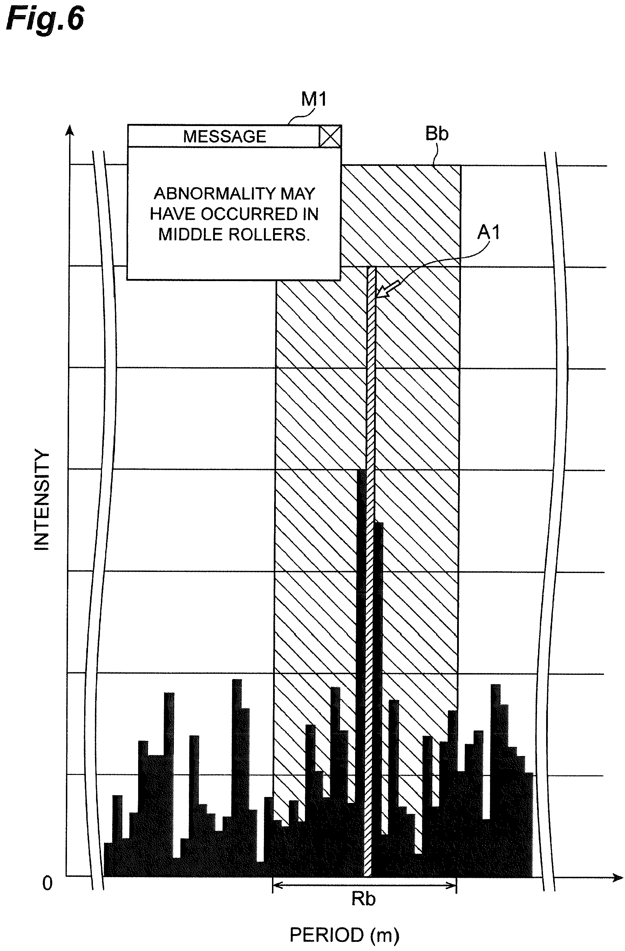

Furthermore, when the specific region Ba, Bb, Bc, or Bd is selected (for example, a pointer A1 is placed thereover as depicted in FIG. 6), the display control unit 60C causes a defect factor, which is highly relevant to periods in a specific range corresponding to each of the specific regions Ba, Bb, Bc, and Bd, to be displayed in a visible manner. For example, as depicted in FIG. 6, when the specific region Bb is selected by the operator, a message M1 saying "abnormality may have occurred in the middle rollers." pops up.

As depicted in FIG. 4, the display control unit 60C controls the display device 52 such that the second bar graph BG2 is displayed in an overlapping manner in a result displaying area R2 having an abscissa AX for representing periodic bands, an ordinate AY for representing peak intensities (intensity) (CV%) (distribution quantities) corresponding to the respective periodic bands, and specific regions Be, Bf, Bg, Bh, and Bi that are regions corresponding to periodic bands in specific ranges and are displayed in a visually distinguishable manner.

For example, it is assumed herein that the following items are known based on empirical rules, theoretical values, and the like.

- When a value equal to or larger than a predetermined threshold appears in a period that belongs to a periodic band range Re, abnormality may have occurred in the front bottom roller 20b.
- When a value equal to or larger than a predetermined threshold appears in a periodic band that belongs to a periodic band range Rf, abnormality may have occurred in the front top roller 20a.
- When a value equal to or larger than a predetermined threshold appears in a periodic band that belongs to a periodic band range Rg, abnormality may have occurred in the middle top roller 19a.
- When a value equal to or larger than a predetermined threshold appears in a periodic band that belongs to a periodic band range Rh, abnormality may have occurred in the bottom apron belt 18b.
- When a value equal to or larger than a predetermined threshold appears in a periodic band that belongs to a periodic band range Ri, abnormality may have occurred in a driving motor (not depicted) for the third bottom roller 17b.

Each threshold herein is a value that is determined in advance for a protruding amount relative to peak intensities in the adjacent periodic bands. Thus, depending on peak intensities in the adjacent periodic bands, even the same value may be determined to be smaller than the threshold.

The display control unit 60C causes the specific regions Be, Bf, Bg, Bh, and Bi to be displayed in colors different from that of the other region (background) B0 excluding the specific regions Be, Bf, Bg, Bh, and Bi. The specific regions Be, Bf, Bg, Bh, and Bi maybe displayed in colors different from each other, or may be displayed in the same color.

Such coloring caused by the display control unit 60C enables the operator to objectively recognize, for example, that abnormality may have occurred in the front bottom roller 20b when the specific region Be containing the periodic band range Re overlaps a bar B that represents a peak intensity exceeding the predetermined threshold. In the same manner, the operator can objectively recognize, for example, that abnormality may have occurred in the front top roller 20a when the specific region Bf containing the periodic band range Rf overlaps a bar B representing a peak intensity exceeding the predetermined threshold, that abnormality may have occurred in the middle top roller 19a when the specific region Bg containing the periodic band range Rg overlaps a bar B representing a peak intensity exceeding the predetermined threshold, that abnormality may have occurred in the bottom apron belt 18b when the specific region Bh containing the periodic band range Rh overlaps a bar B representing a peak intensity exceeding the predetermined threshold, and that abnormality may have occurred in the driving motor for the third bottom roller 17b when the specific region Bi containing the periodic band range Ri overlaps a bar B representing a peak intensity exceeding the predetermined threshold.

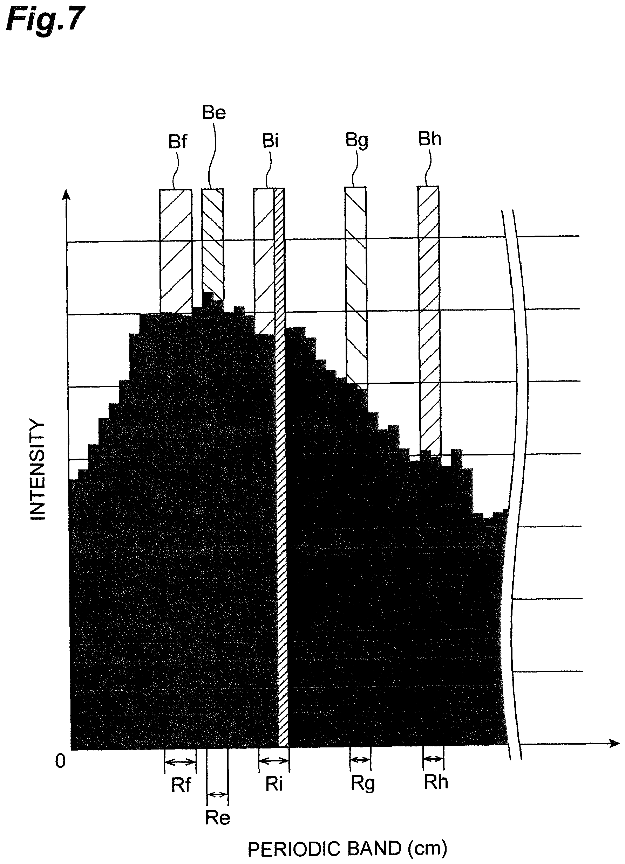

Furthermore, the display control unit 60C causes a bar B the peak intensity of which exceeds the predetermined threshold to be displayed in a manner visually distinguishable from bars B the peak intensities of which do not exceed the predetermined thresholds. For example, as depicted in FIG. 7, the display control unit 60C causes a bar B the peak intensity of which exceeds the predetermined threshold to be displayed in a color (hatched in FIG. 7) different from that of bars B the peak intensities of which do not exceed the predetermined thresholds.

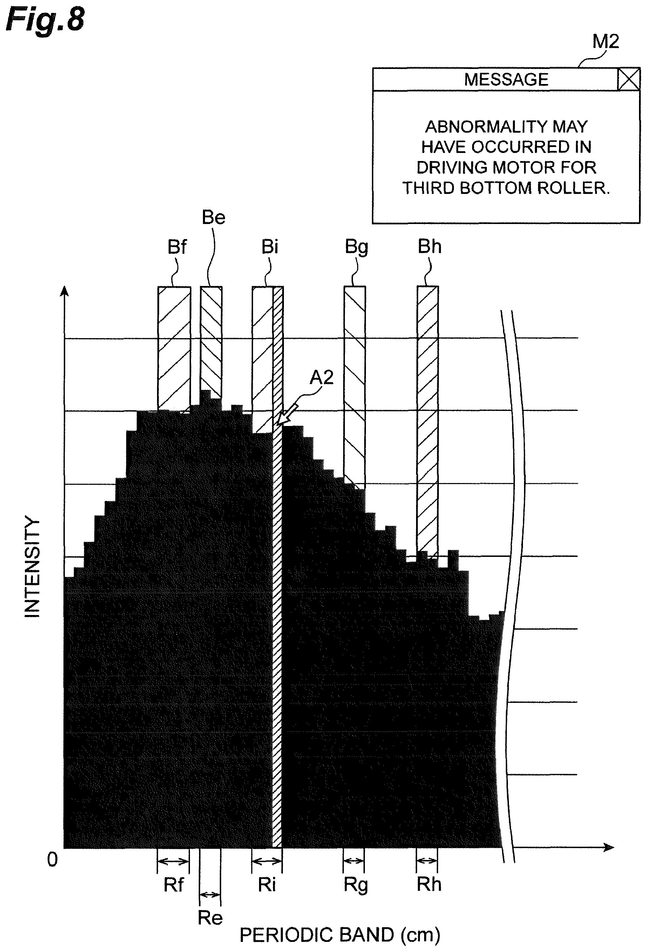

Furthermore, the specific region Be, Bf, Bg, Bh, or Bi is selected (for example, a pointer A2 is placed thereover as depicted in FIG. 8), the display control unit 60C causes information, which is relevant to a part to become a factor of abnormality that occurs in periodic bands in a specific range corresponding to each of the specific regions Be, Bf, Bg, Bh, and Bi, to be displayed in a visible manner. For example, as depicted in FIG. 8, when the specific region Bi is selected by a user, a message M2 saying "abnormality may have occurred in the driving motor for the third bottom roller." pops up.

The display control unit 60C may cause the first bar graph BG1 and the second bar graph BG2 described above to be displayed for each spinning unit 2, that is, for each spindle, or to be displayed for a plurality of spindles. Alternatively, by operation of the operator, data of a spindle to be displayed may be selected. Furthermore, when abnormality has occurred in a spindle (when the peak intensity of each bar exceeds the predetermined threshold), the display control unit 60C may cause the first bar graph BG1 and the second bar graph BG2 for the spindle in which abnormality has occurred to be displayed with a higher priority.

The display control unit 60C can cause a screen to be displayed so as to be selectively switched between a screen displaying the first bar graph BG1 depicted in FIG. 3 and a screen displaying the second bar graph BG2 depicted in FIG. 4. The first bar graph BG1 and the second bar graph BG2 may be displayed on a single screen.

The following describes functional effects of the analyzing device 60 according to the present embodiment. As depicted in FIG. 3, in the analyzing device 60 configured as described above, the first bar graph BG1 representing with the bars B the relation between periods and peak intensities that is analyzed by the analyzing unit 60B is displayed in the result displaying area R1 having the specific regions Ba, Bb, Bc, and Bd that are regions corresponding to the specific periodic ranges Ra, Rb, Rc, and Rd and are displayed in a visually distinguishable manner. This enables the operator to determine, at a glance, to which periodic region a period the peak intensity of which projects corresponds. As depicted in FIG. 4, in the analyzing device 60 configured as described above, the second bar graph BG2 representing with the bars B the relation between periodic bands and peak intensities that is analyzed by the analyzing unit 60B is displayed in the result displaying area R2 having the specific regions Be, Bf, Bg, Bh, and Bi that are regions corresponding to the specific periodic band ranges Re, Rf, Rg, Rh, and Ri and are displayed in a visually distinguishable manner. This enables the operator to determine, at a glance, to which periodic band region a periodic band the peak intensity of which projects corresponds.

When the specific regions Ba, Bb, Bc, and Bd or the specific regions Be, Bf, Bg, Bh, and Bi are each displayed in different display modes (e. g. , different colors), the thresholds for changing the display modes at occurrence of abnormality do not have to be determined in advance. Displaying the specific regions Ba, Bb, Bc, and Bd or the specific regions Be, Bf, Bg, Bh, and Bi each in different colors can help the operator so that the operator can view the height of each bar B and the period or the periodic band to which the bar B belongs by himself/herself and can determine the presence or absence of abnormality, a cause of the abnormality, and the level of the abnormality by himself/herself. The operator can also recognize, for example, a situation in which abnormality is likely to occur soon even if abnormality has not yet occurred.

In the present embodiment, as depicted in FIG. 3, the specific regions Ba, Bb, Bc, and Bd are displayed so as to be respectively associated with abnormal parts (the front rollers 20, the middle rollers 19, the third rollers 17, and the back rollers 16) derived fromempirical rules, theoretical values, and the like. Thus, for example, if the operator identifies that a bar B the peak intensity of which projects belongs to the specific region Bd, the operator can objectively recognize that abnormality may have occurred in the back rollers 16. Consequently, regardless of differences in experience among operators, the abnormal part can be identified.

In the present embodiment, as depicted in FIG. 4, the specific regions Be, Bf, Bg, Bh, and Bi are displayed so as to be respectively associated with abnormal parts (the front bottom roller 20b, the front top roller 20a, the middle top roller 19a, the bottom apron belt 18b, and the driving motor for the third bottom roller 17b) derived from empirical rules, theoretical values, and the like. Thus, for example, if the operator identifies that a bar B the peak intensity of which projects belongs to the specific region Bi, the operator can objectively recognize that abnormality may have occurred in the driving motor for the third bottom roller 17b. Consequently, regardless of differences in experience among operators, the abnormal part can be identified.

In the present embodiment, the display control unit 60C causes the specific regions Ba, Bb, Bc, and Bd to be displayed in colors different from that of the other region (background) B0, which enables the operator to easily distinguish the specific regions Ba, Bb, Bc, and Bd. In the same manner, the display control unit 60C causes the specific regions Be, Bf, Bg, Bh, and Bi to be displayed in colors different from that of the other region (background) B0, which enables the operator to easily distinguish the specific regions Be, Bf, Bg, Bh, and Bi.

The display control unit 60C causes a bar B representing a peak intensity to be displayed for each period. Specifically, the first bar graph BG1 is caused to be displayed. Thus, data for identifying a mechanical factor can be provided.

The display control unit 60C causes a bar B representing a peak intensity to be displayed for each periodic band. Specifically, the second bar graph BG2 is caused to be displayed. For example, even if no damage exists in the drafting rollers 16, 17, 19, and 20, due to properties (average fiber length or distribution of fiber length, etc.) of the sliver S, traveling of part of a fiber in the sliver S traveling between the drafting rollers 16, 17, 19, and 20 may fluctuate, so that irregularity (yarn irregularity) may occur in the yarn Y thus spun (for example, yarn irregularity may occur when the drafting rollers 16, 17, 19, and 20 are disposed at pitches each of which is longer than the average fiber length of the sliver S). Such yarn irregularity does not appear as an abnormal value in peak intensity for each period, but may appear as an abnormal value when the peak intensity is calculated for each periodic band. In the present embodiment, data for identifying a factor derived from yarn physical properties as described above can be provided.

One embodiment of the present disclosure has been described in the foregoing, the present disclosure is not limited to the embodiment.

<Modification 1>

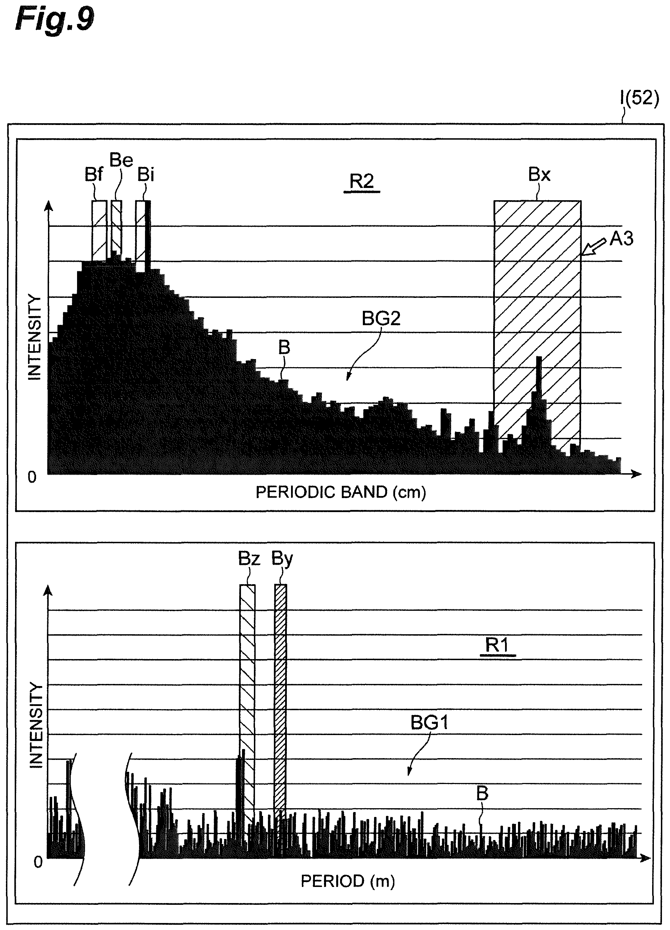

As depicted in FIG. 9, a display control unit 60C according to a modification 1 may perform control such that the second bar graph BG2 (see FIG. 4) representing a peak intensity for each frequency band or each periodic band and the first bar graph BG1 (see FIG. 3) representing a peak intensity for each frequency or each period are displayed on one screen I of the display device 52.

Furthermore, for example, the display control unit 60C may cause the second bar graph BG2 to be displayed in the result displaying area (second result displaying area) R2, and may cause the first bar graph BG1 to be displayed in the result displaying area (first result displaying area) R1 such that, when one of the specific regions Be, Bf, Bi, and Bx in the second bar graph BG2 is selected, one of the corresponding regions (specific regions) in the first bar graph BG1 that are each associated with the specific regions Be, Bf, Bi, and Bx becomes visually distinguishable. Herein, although the term "corresponding regions" in a different name is used in a sense that each region is associated with one of the specific regions contained in the result displaying area R in which the first bar graph BG1 is displayed, the corresponding regions are not substantially different in character from the respective specific regions Be, Bf, Bi, and Bx. In this case, as the first bar graph BG1, the first bar graph BG1 for all periods may be displayed, or only the first bar graph BG1 for periods that are relevant to the selected specific region of the second bar graph BG2 may be displayed. The first bar graph may be displayed in which the first bar graph BG1 for periods that are irrelevant to the selected specific region of the second bar graph BG2 is omitted appropriately (in consideration of the size or easy viewability of the screen, for example).

The number of corresponding regions in the first bar graph BG1 that are each associated with the specific regions Be, Bf, Bi, and Bx may be one or may be two or more. When one of the specific regions in the first bar graph BG1 is selected, the display control unit 60C may causes the second bar graph to be displayed such that one of the corresponding regions in the second bar graph BG2 that are each associated with the specific regions becomes visually distinguishable.

Specific description will be made below. For example, it is assumed herein that the following items are known based on empirical rules, theoretical values, and the like. It is also assumed that corresponding regions By and Bz in the first bar graph BG1 are associated with a specific region Bx in the second bar graph BG2.

- When abnormality has occurred in a frequency band in the specific region Bx of the second bar graph BG2, abnormality due to at least one of a mechanical factor and yarn physical properties is highly likely to have occurred between the middle rollers 19 and the third rollers 17.

- When a value equal to or larger than a predetermined threshold appears in a periodic range Ry contained in the corresponding region By, abnormality is highly likely to have occurred in the bottom apron belt 18b.

- When a value equal to or larger than a predetermined threshold appears in a periodic range Rz contained in the corresponding region Bz, abnormality is highly likely to have occurred in the middle top roller 19a.

For example, if a value equal to or larger than a predetermined threshold appears in a periodic band belonging to the periodic band range Rx of the second bar graph BG2, this enables the operator to objectively recognize that abnormality due to at least one of a mechanical factor and yarn physical properties is highly likely to have occurred between the middle rollers 19 and the third rollers 17. Herein, when the operator moves a pointer A3, for example, to select the specific region Bx containing the periodic band range Rx, the corresponding region By and the corresponding region Bz in the first bar graph BG1 that are associated with the specific region Bx are displayed in colors. This enables the operator to focus on values of bars B in the corresponding regions By and Bz displayed in colors.

Herein, for example, when a value equal to or larger than the predetermined threshold does not appear in a bar B in the periodic range Ry contained in the corresponding region By, the operator can objectively recognize that there is no possibility of abnormality in the bottom apron belt 18b. For example, when a value equal to or larger than the predetermined threshold appears in the periodic range Rz contained in the corresponding region Bz, the operator can objectively recognize that abnormality may have occurred in the middle top roller 19a. For example, when a projecting value appears in frequency bands in the specific region Bx but a value equal to or larger than the predetermined threshold does not appear in either of the periodic range Ry and the periodic range Rz, the operator can objectively recognize that such a value is highly likely to appear in the frequency bands in the specific region Bx due to yarn physical properties.

As described above, with the analyzing device 60 according to the modification 1, the operator can determine the abnormal part from a comprehensive viewpoint considering two factors of a mechanical factor and a factor derived from yarn physical properties. Occurrence of abnormality that is difficult to be recognized by viewing only either one of the first bar graph BG1 and the second bar graph BG2 can be recognized by viewing both of the first bar graph BG1 and the second bar graph BG2.

If the number of abnormality locations where the predetermined thresholds are exceeded in the second bar graph BG2 is one, the region on the first bar graph BG1 side corresponding to the specific region that belongs to the corresponding abnormality location may be automatically displayed in color.

<Other Modifications>

In the embodiment or the modification above, examples have been described in which the first bar graph BG1 using the period for the abscissa AX is displayed on the display device 52, and the second bar graph BG2 using the periodic band is displayed on the display device 52. However, the frequency or the frequency band may be used for the abscissa AX.

In the embodiment or the modification above, examples have been described in which the display control unit 60C causes the specific regions Ba, Bb, Bc, Bd, Be, Bf, Bg, Bh, Bi, and Bx and the corresponding regions By and Bz to be displayed in colors. However, these regions maybe displayed in any displaymode if they are visually distinguishable and, for example, may be hatched, patterned, or displayed in other modes. Coloring of the specific regions Ba, Bb, Bc, Bd, Be, Bf, Bg, Bh, Bi, and Bx and the corresponding regions By and Bz may be performed by differentiating hue, brightness, and a combination of hue and brightness from the background.

In the embodiment or the modification above, examples have been described in which, when one of the specific regions Ba, Bb, Bc, Bd, Be, Bf, Bg, Bh, Bi, and Bx and the corresponding regions By and Bz is selected by the user, the display control unit 60C receives this selection and causes information on an abnormal part that is highly relevant to the corresponding specific region to pop up. However, the present disclosure is not limited to this. For example, in anywhere on the display device 52, a legend or the like indicating a relation between each of the specific regions Ba, Bb, Bc, Bd, Be, Bf, Bg, Bh, Bi, and Bx and the corresponding regions By and Bz and the corresponding abnormal part may be displayed. Information on the abnormal parts may be displayed near the positions of the specific regions Ba, Bb, Bc, Bd, Be, Bf, Bg, Bh, Bi, and Bx and the corresponding regions By and Bz.

In the embodiment and the modification above, examples have been described in which the display control unit 60C controls the display device 52 such that a portion in which the relative difference (protruding amount) in distribution quantity with respect to the distribution quantities in the adjacent frequency bands or the adjacent periodic bands exceeds a predetermined threshold becomes visually distinguishable from a portion in which the relative difference does not exceed the predetermined threshold. However, the present disclosure is not limited to this. For example, the display control unit may control the display device 52 such that a portion in which the absolute value of the distribution quantity of a frequency band or a periodic band exceeds a predetermined value becomes visually distinguishable from a portion in which the absolute value does not exceed the predetermined value.

All of the backgrounds of the bar graphs (the first bar graph BG1 and the second bar graph BG2) may be displayed in the same displaymode (e.g., in the same color) . When a bar B exceeds a threshold, the bar B may be displayed in a color that is determined in advance for a region to which the bar B exceeding the threshold belongs. For example, a bar graph for each period may be displayed in an area in which a plurality of specific regions are displayed in a visually distinguishable manner (displayed in colors) in the background as in the embodiment described above, and all the background of a bar graph for each periodic band may be displayed in the same display mode (e.g., in the same color). When a bar B exceeds a threshold, the bar B may be displayed in a color that is determined in advance for a region to which the bar B exceeding the threshold belongs. All the background of a bar graph for each period may be displayed in the same display mode (e.g., in the same color). When a bar B exceeds a threshold, the bar B may be displayed in a color that is determined in advance for a region to which the bar B exceeding the threshold belongs. A bar graph for each periodic band may be displayed in an area in which a plurality of specific regions are displayed in a visually distinguishable manner (displayed in colors) in the background as in the embodiment described above.

In the bar graphs, examples have been described in which a bar B exceeding a predetermined threshold is displayed in a color that is different from that of bars B the peak intensities of which do not exceed the predetermined threshold (hatched in FIG. 7). However, for example, the bar B may be displayed in "blue under normal conditions, yellow if exceeding a first threshold, and red if exceeding a second threshold". In other words, a plurality of thresholds may be set for a single bar B, and different display modes may be used for a plurality of settings. In the result displaying area R1 in which the bar graph is displayed, graphs each representing the first threshold and the second threshold may be displayed in display modes different from each other (e.g., with a yellow continuous line and a red continuous line). Herein, an example has been described in which the color of the graph representing the first threshold and the color of the bar B exceeding the first threshold are the same, and the color of the graph representing the second threshold and the color of the bar B exceeding the second threshold are the same. However, the present disclosure is not limited to this.

The analyzing device 60 may be configured to be able to display only the first bar graph BG1, or may be configured to be able to display only the second bar graph BG2.

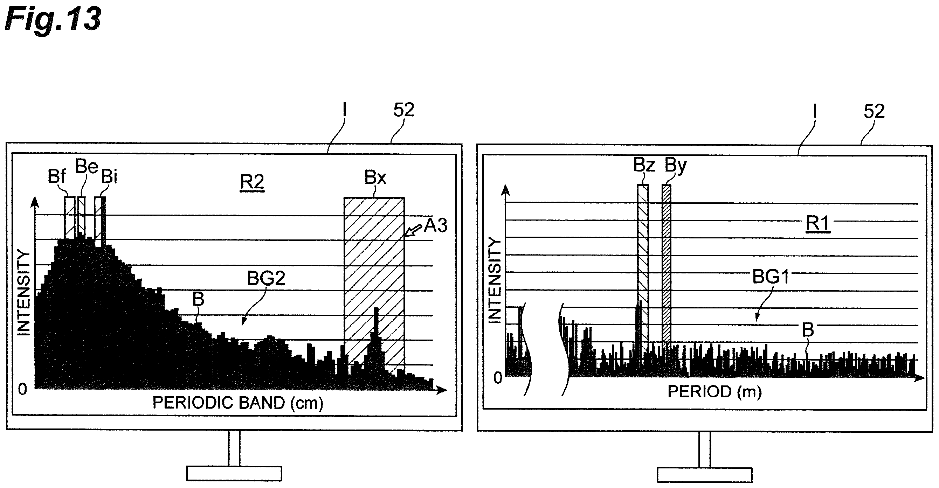

Coordinated display of the first bar graph BG1 and the second bar graph BG2 as described in the modification 1 is not limited to an example in which these bar graphs are displayed on one screen of one display device 52 as depicted in FIG. 9. The coordinated display may be performed by switching between the screen displaying the first bar graph BG1 and the screen displaying the second bar graph BG2 on one screen of the display device 52. Alternatively, as depicted in FIG. 13, the coordinated display may be performed by providing two display devices 52, displaying the first bar graph BG1 on one display device 52, and displaying the second bar graph BG2 on the other display device 52.

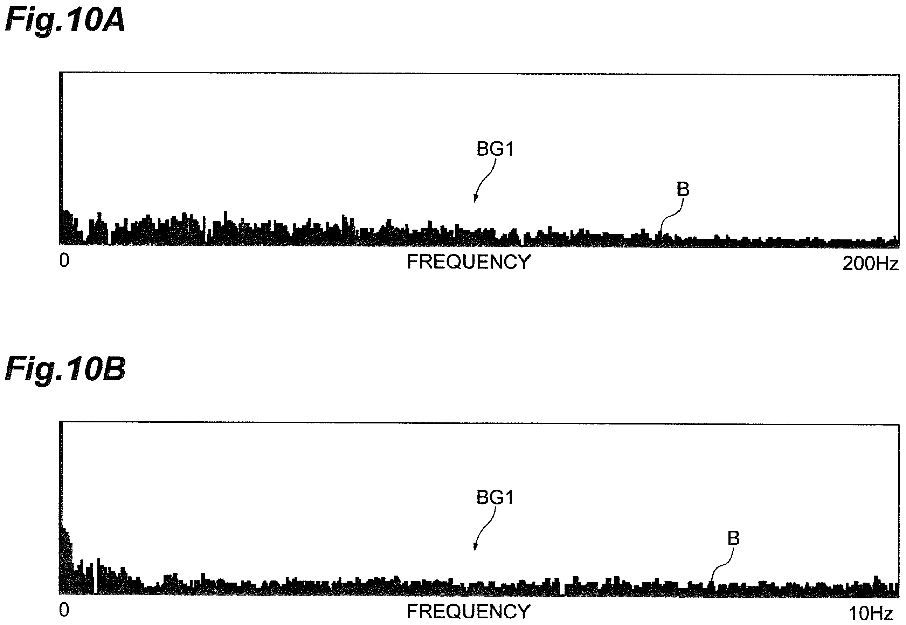

The first bar graph having the abscissa representing the period or the frequency may be displayed so as to be divided into two graphs, one for short periods in which the range of periods in the abscissa is relatively short, and one for long periods in which the range of periods in the abscissa is relatively long. For example, the first bar graph may be displayed so as to be divided into two graphs of the first bar graph BG1 as depicted in FIG. 10A in which the range of frequencies in the abscissa is 0 Hz to 200 Hz and the first bar graph BG1 as depicted in FIG. 10B in which the range of frequencies in the abscissa is 0 Hz to 10 Hz. By this displaying, for example, when the specific regions described above are positioned very close to one another within a predetermined range, the individual specific regions seem to be overlap each other and are difficult to be visually identified in the first bar graph BG1 depicted in FIG. 10A, whereas the specific regions are displayed so as to be spaced apart from each other in the first bar graph BG1 depicted in FIG. 10B. This facilitates the individual specific regions to be visually identified. The range of frequencies in the first bar graph BG1 in which the range in the abscissa is narrow is not limited to the range of 0 Hz to 10 Hz as depicted in FIG. 10B, and may be an optional range. Similar displaying may be used for the second bar graph BG2.

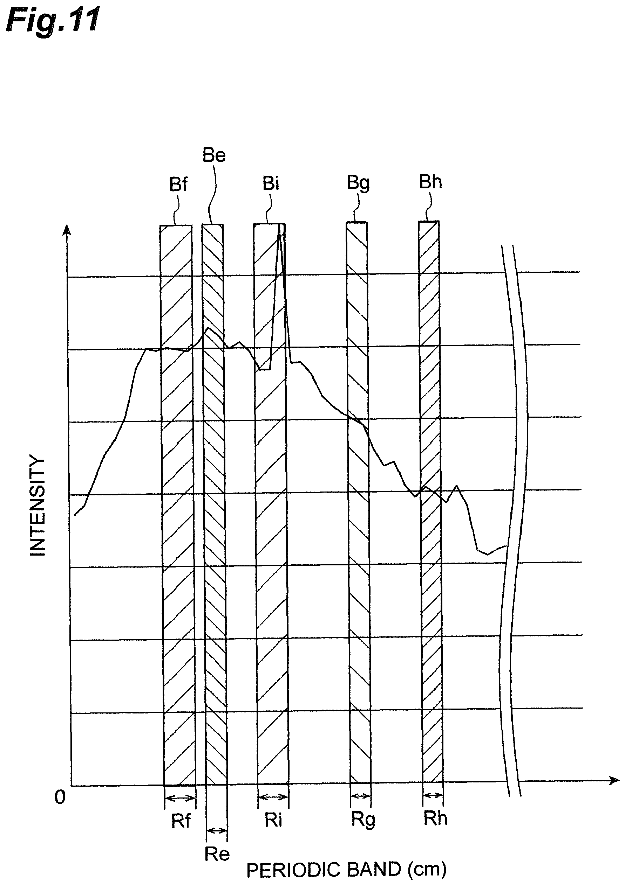

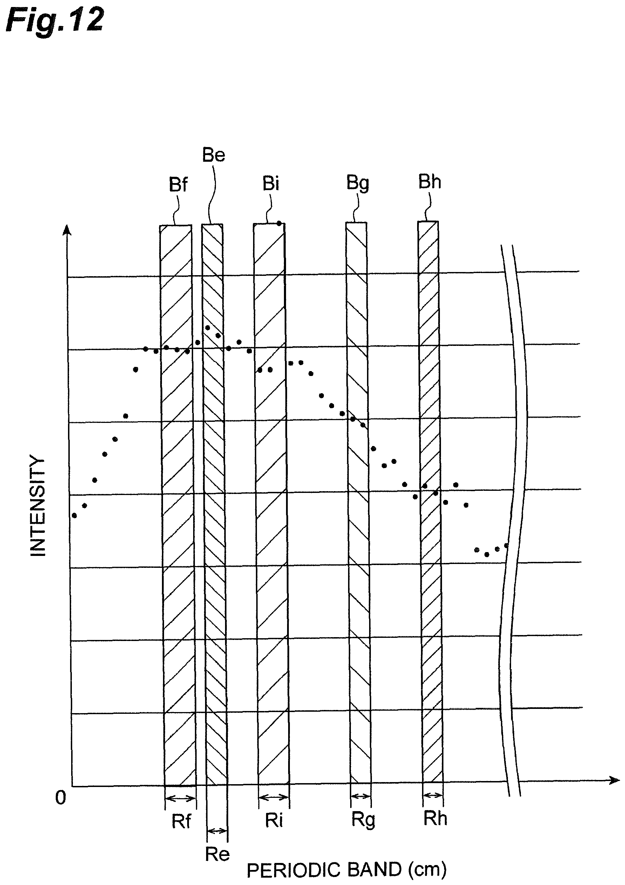

In the embodiment or the modifications above, examples have been described in which a relation between frequencies (frequency bands) or periods (periodic bands) and distribution quantities that is analyzed by the analyzing unit 60B is displayed as a bar graph. However, for example, the relation between frequencies (frequency bands) or periods (periodic bands) and distribution quantities may be displayed as a line graph as depicted in FIG. 11, or may be displayed as a dot matrix as depicted in FIG. 12. The line graph and the dot matrix may be displayed in an overlapping manner.

In such a modification, the display control unit 60C may control the display device 52 such that, for example, a dot that exceeds a predetermined threshold becomes visually distinguishable from dots that do not exceed the predetermined threshold. In this modification, the operator can be effectively notified of a location where a problem may have occurred.

In the embodiment or the modifications above, examples have been described in which the results of analysis performed by the analyzing device 60 are displayed on the display device 52 of the second end frame 5, but the present disclosure is not limited to this. For example, the analysis result may be displayed on a display device (not depicted) provided on the spinning unit 2 side, or may be displayed on another server, a personal computer, a smart phone, or the like that is accessibly connected via a wired or wireless LAN, for example. The analyzing device 60 does not have to directly control the display device 52, another server, a personal computer, a smart phone, or the like that is accessibly connected via a wired or wireless LAN, for example, and may be configured to only generate data (e.g., image data) that can be displaced on these display devices.

In the embodiment or the modifications above, examples have been described in which the result displaying area R1 (R2) has a plurality of specific regions, but the result displaying area may have only one specific region.

At least some parts of the embodiment and the modifications described above may be optionally used in combination.

**Claims**

1. An analyzing device (60) configured to analyze yarn (Y) to be processed in a fiber processing unit (2) based on yarn thickness information on thickness of the yarn (Y), the analyzing device (60) comprising:

   an acquiring unit (60A) configured to acquire the yarn thickness information;

   an analyzing unit (60B) configured to analyze time variation of the yarn thickness based on the yarn thickness information acquired by the acquiring unit (60A); and

   a display control unit (60C) configured to control a display unit (52) to display at least one graph (BG1, BG2) in a result displaying area (R1, R2) having one of an abscissa (AX) and an ordinate (AY) for representing a frequency or a period in which a predetermined yarn thickness appears, the other of the abscissa (AX) and the ordinate (AY) for representing a distribution quantity of the frequency or the period, and at least one specific region (Ba, Bb, Bc, Bd, Be, Bf, Bg, Bh, Bi, Bx, By, Bz) in which a region corresponding to the frequency or the period in a specific range is displayed in a visually distinguishable manner, the graph (BG1, BG2) representing a relation between the frequency or the period and the distribution quantity, the relation being analyzed by the analyzing unit (60B).

2. The analyzing device (60) according to claim 1, wherein the display control unit (60C) controls the display unit (52) so as to display at least one of the graph (BG1, BG2) representing the distribution quantity for each frequency or each period and the graph (BG1, BG2) representing the distribution quantity for each frequency band that is a predetermined range of the frequency or for each periodic band that is a predetermined range of the period.

3. The analyzing device (60) according to claim 2, wherein the display control unit (60C) controls the display unit (52) such that a portion in which the distribution quantity exceeds a predetermined threshold becomes visually distinguishable from a portion in which the distribution quantity does not exceed the predetermined threshold.

4. The analyzing device (60) according to claim 2, wherein the display control unit (60C) controls the display unit (52) such that a portion in which the distribution quantity exceeds a predetermined threshold with respect to the distribution quantity in an adjacent frequency band or an adjacent periodic band becomes visually distinguishable from a portion in which the distribution quantity does not exceed the predetermined threshold.

5. The analyzing device (60) according to any one of claims 2 to 4, wherein the display control unit (60C) receives selection of the specific region (Ba, Bb, Bc, Bd, Be, Bf, Bg, Bh, Bi, Bx, By, Bz) made by operation of an operator.

6. The analyzing device (60) according to claim 5, wherein the display control unit (60C) controls the display unit (52) so as to display a first result displaying area (R1) and a second result displaying area (R2) as the result displaying area (R1, R2), display a first graph (BG1) representing the distribution quantity for each frequency or each period in the first result displaying area (R1), display a second graph (BG2) representing the distribution quantity for each frequency band or each periodic band in the second result displaying area (R2), and having received selection of one specific region (Bx) contained in the result displaying area (R1, R2) in which one of the first graph (BG1) and the second graph (BG2) is displayed, make visually distinguishable at least one specific region (By,Bz) contained in the result displaying area (R1, R2) in which the other of the first graph (BG1) and the second graph (BG2) is displayed, the at least one specific region (By,Bz) being associated with the one specific region (Bx).

7. The analyzing device (60) according to claim 5 or 6, wherein the display control unit (60C) controls the display unit (52) such that, when selection of the specific region (Ba, Bb, Bc, Bd, Be, Bf, Bg, Bh, Bi, Bx, By, Bz) has been received, information on a part to become a factor of abnormality that occurs in the frequency or the period in the specific range corresponding to the specific region (Ba, Bb, Bc, Bd, Be, Bf, Bg, Bh, Bi, Bx, By, Bz) becomes visually distinguishable.

8. The analyzing device (60) according to any one of claims 2 to 7, wherein the graph (BG1, BG2) is a bar graph (BG1, BG2).

9. The analyzing device (60) according to claim 8, wherein the display control unit (60C) controls the display unit (52) so as to display a bar (B) of the bar graph (BG1, BG2) representing the distribution quantity for each frequency or each period.

10. The analyzing device (60) according to claim 8 or 9, wherein the display control unit (60C) controls the display unit (52) such that the bar (B) having a distribution quantity exceeding a predetermined threshold becomes visually distinguishable from a bar (B) having a distribution quantity not exceeding the predetermined threshold.

11. The analyzing device (60) according to claim 8, wherein the display control unit (60C) controls the display unit (52) so as to display a bar (B) of the bar graph (BG1, BG2) representing the distribution quantity for each frequency band or each periodic band.

12. The analyzing device (60) according to claim 11, wherein the display control unit (60C) controls the display unit (52) such that the bar (B) having a protruding amount relative to the distribution quantity in an adjacent frequency band or an adjacent periodic band exceeding a predetermined threshold becomes visually distinguishable from the bar (B) having a protruding amount not exceeding the predetermined threshold.

13. The analyzing device (60) according to any one of claims 1 to 12, wherein the display control unit (60C) controls the display unit (52) such that the specific region (Ba, Bb, Bc, Bd, Be, Bf, Bg, Bh, Bi, Bx, By, Bz) is displayed in a display mode different from that of the other region.

14. The analyzing device (60) according to claim 13, wherein the display control unit (60C) controls the display unit (52) such that the specific region (Ba, Bb, Bc, Bd, Be, Bf, Bg, Bh, Bi, Bx, By, Bz) is displayed in a color different from that of the other region.

15. The analyzing device (60) according to any one of claims 1 to 14, wherein the specific region (Ba, Bb, Bc, Bd, Be, Bf, Bg, Bh, Bi, Bx, By, Bz) includes at least a region corresponding to a frequency or a period corresponding to a rotation period of a front top roller (20a) that is calculated based on a radius of the front top roller (20a) and a rotation speed of the front top roller (20a).

16. A spinning machine (1) comprising:

a drafting device (6) configured to draft a fiber bundle (F);
an air spinning device (7) configured to generate yarn (Y) by twisting the fiber bundle (F) drafted by the drafting device (6);
a winding device (13) configured to wind the yarn (Y) generated by the air spinning device (7) to form a package (P);
the analyzing device (60) according to any one of claims 1 to 15; and
a display unit (52), wherein
the display unit (52) displays a graph (BG1, BG2) in the result displaying area (R1, R2), the graph (BG1, BG2) representing a relation between the frequency or the period and the distribution quantity, the relation being analyzed by the analyzing unit (60B).

*Fig.1*

EP 3 276 348 A1

# Fig.2

1

```
┌─────────────────────────────────────────────┐
│                              51               │
│        MACHINE CONTROL DEVICE                 │
│   ┌─────────────────────────────────┐  60    │
│   │       ANALYZING DEVICE           │        │
│   │   ┌─────────────────────────┐ 60A│        │
│   │   │    ACQUIRING UNIT       │    │        │
│   │   └─────────────────────────┘    │        │
│   │   ┌─────────────────────────┐ 60B│        │
│   │   │    ANALYZING UNIT       │    │        │
│   │   └─────────────────────────┘    │        │
│   │   ┌─────────────────────────┐ 60C│        │
│   │   │       DISPLAY           │    │        │
│   │   │    CONTROL UNIT         │    │        │
│   │   └─────────────────────────┘    │        │
│   └─────────────────────────────────┘        │
```

YARN MONITORING DEVICE — 8

UNIT CONTROLLER — 10

ANALYZING DEVICE — 60

ACQUIRING UNIT — 60A

ANALYZING UNIT — 60B

DISPLAY CONTROL UNIT — 60C

DISPLAY DEVICE — 52

INPUT KEY — 53

15

EP 3 276 348 A1

Fig.3

*Fig.4*

**Fig.5**

# Fig.6

# Fig.7

# Fig.8

M2

MESSAGE

ABNORMALITY MAY
HAVE OCCURRED IN
DRIVING MOTOR FOR
THIRD BOTTOM ROLLER.

INTENSITY

Bf   Be   Bi   Bg   Bh

A2

Rf   Re   Ri   Rg   Rh

PERIODIC BAND (cm)

*Fig.9*

## Fig.10A

## Fig.10B

EP 3 276 348 A1

*Fig.11*

## Fig.12

EP 3 276 348 A1

# Fig.13

Europäisches
Patentamt

European
Patent Office

Office européen
des brevets

## EUROPEAN SEARCH REPORT

**Application Number**

EP 17 18 2970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/061359 A1 (MEIXNER CHRISTINE [CH] ET AL) 17 March 2011 (2011-03-17) * abstract; figures 5-11 * ----- | 1-16 | INV. G01N33/36 |
| X | CH 699 219 A1 (USTER TECHNOLOGIES AG [CH]) 29 January 2010 (2010-01-29) * abstract; figures 2-5 * ----- | 1-5,7, 13-16 | |

| | | | |
|---|---|---|---|
| | | | **TECHNICAL FIELDS SEARCHED (IPC)** |
| | | | G01N |

The present search report has been drawn up for all claims

| Place of search | Date of completion of the search | Examiner |
|---|---|---|
| The Hague | 21 December 2017 | Wilhelm-Shalganov, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 18 2970

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2017

| Patent document cited in search report | | Publication date | Patent family member(s) | | Publication date |
|---|---|---|---|---|---|
| US 2011061359 | A1 | 17-03-2011 | CN | 101310180 A | 19-11-2008 |
| | | | CN | 101310181 A | 19-11-2008 |
| | | | CN | 103590230 A | 19-02-2014 |
| | | | CN | 103591918 A | 19-02-2014 |
| | | | CN | 103592427 A | 19-02-2014 |
| | | | EP | 1949096 A2 | 30-07-2008 |
| | | | EP | 1949097 A2 | 30-07-2008 |
| | | | EP | 1960770 A2 | 27-08-2008 |
| | | | EP | 2270494 A2 | 05-01-2011 |
| | | | EP | 2275813 A2 | 19-01-2011 |
| | | | EP | 2278328 A2 | 26-01-2011 |
| | | | JP | 5077772 B2 | 21-11-2012 |
| | | | JP | 5354343 B2 | 27-11-2013 |
| | | | JP | 5354344 B2 | 27-11-2013 |
| | | | JP | 5354407 B2 | 27-11-2013 |
| | | | JP | 5354408 B2 | 27-11-2013 |
| | | | JP | 5354409 B2 | 27-11-2013 |
| | | | JP | 2009516783 A | 23-04-2009 |
| | | | JP | 2009516784 A | 23-04-2009 |
| | | | JP | 2009516785 A | 23-04-2009 |
| | | | JP | 2012132139 A | 12-07-2012 |
| | | | JP | 2012132140 A | 12-07-2012 |
| | | | JP | 2012136819 A | 19-07-2012 |
| | | | US | 2008230728 A1 | 25-09-2008 |
| | | | US | 2008276593 A1 | 13-11-2008 |
| | | | US | 2008288101 A1 | 20-11-2008 |
| | | | US | 2011061358 A1 | 17-03-2011 |
| | | | US | 2011061359 A1 | 17-03-2011 |
| | | | WO | 2007056883 A2 | 24-05-2007 |
| | | | WO | 2007056884 A2 | 24-05-2007 |
| | | | WO | 2007056885 A2 | 24-05-2007 |
| CH 699219 | A1 | 29-01-2010 | CH | 699219 A1 | 29-01-2010 |
| | | | CN | 101970326 A | 09-02-2011 |
| | | | EP | 2303743 A1 | 06-04-2011 |
| | | | JP | 5257800 B2 | 07-08-2013 |
| | | | JP | 2011529016 A | 01-12-2011 |
| | | | WO | 2010009565 A1 | 28-01-2010 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007224452 A